Europäisches Patentamt

(19) European Patent Office

Office européen des brevets

(11) Publication number: **0 243 174**
**A2**

(12) **EUROPEAN PATENT APPLICATION**

(21) Application number: **87303543.0**

(22) Date of filing: **22.04.87**

(51) Int. Cl.⁴: **C 12 P 21/00**
**C 12 N 15/00, A 61 K 39/40,**
**G 01 N 33/577**

(30) Priority: **22.04.86 US 855005**

(43) Date of publication of application:
**28.10.87 Bulletin 87/44**

(84) Designated Contracting States:
**AT BE CH DE ES FR GB GR IT LI LU NL SE**

(71) Applicant: **CETUS CORPORATION**
**1400 Fifty-Third Street**
**Emeryville California 94608 (US)**

**OREGON HEALTH SCIENCES UNIVERSITY**
**3181 S.W. Sam Jackson Park Road**
**Portland Oregon 97201 (US)**

(72) Inventor: **Markowitz, Avi B.**
**1821 Monterey Drive**
**San Bruno California 94066 (US)**

**Fendly, Brian**
**49 Showers Drive, J225**
**Mountain View California 94040 (US)**

**Iglewski, Barbara**
**5105 Dudley Lane, 303**
**Bethesda Maryland 20814 (US)**

**Larrick, James**
**Star Route Box 48, 13**
**Woodside California 94062 (US)**

(74) Representative: **Biziey, Richard Edward et al**
**BOULT, WADE & TENNANT 27 Furnival Street**
**London EC4A 1PQ (GB)**

(54) **Monoclonal antibodies to Pseudomonas aeruginosa exoenzyme S, their preparation and use.**

(57) Monoclonal antibodies to P. aeruginosa exoenzyme S are prepared from hybrid cell lines. The antibodies may be of any isotype and may be of any origin such as murine or human origin. Antibodies that exhibit the ability to neutralize the adverse biological effects of the exoenzyme may be used to treat infections caused by P. aeruginosa.

EP 0 243 174 A2

**Description**

MONOCLONAL ANTIBODIES TO PSEUDOMONAS AERUGINOSA EXOENZYME S, THEIR PREPARATION
AND USE

Description

Technical Field

This invention is in the field of immunology. More particularly it concerns monoclonal antibodies to Pseudomonas aeruginosa exoenzyme S, their preparation and use.

Background

P. aeruginosa is a highly virulent pathogen which infects patients receiving immunosuppressive therapy or suffering from severe thermal burns or other serious injuries, cystic fibrosis, or neoplastic diseases. Mortality from P. aeruginosa has been reduced by using agents such as mafenide acetate and silver salts which inhibit bacterial colonization of the burn wound surface, potent antibiotics for treating bacteremia and barrier isolation to minimize contact of the patient with infectious elements. Such agents, however, have only proved partially successful in controlling the morbidity and mortality associated with Pseudomonas infections.

It is not yet clear what components of P. aeruginosa are responsible for its virulence. One of the most extensively studied components of P. aeruginosa is exotoxin A. (Iglewski et al, Methods Enzymol 60:780-793 (1979)). Exotoxin A is on a weight basis the most potent extracellular protein of P. aeruginosa and is produced by about 90% of clinical isolates regardless of Fisher-Devlin-Gnabasik immunotype.

Exoenzyme S is another component that is involved in the virulence of P. aeruginosa. Animal studies with an exoenzyme S-deficient mutant indicate that exoenzyme S is an important virulence factor responsible for the dissemination of bacteria during infection. Exoenzyme S is not as well characterized as exotoxin A, but has recently been purified in two forms. One form is a peptide of molecular weight 49,000 which has adenosine diphosphate ribosyl tranferase activity. The other, a peptide of molecular weight 53,000, is serologically related (antisera react with it and the 49 kd species) but essentially enzymatically inactive, having enzymatic activity at only the 0.1% level of the 49 kd species.

Monoclonal antibodies specific to P. aeruginosa are described in the art. Hancock et al, Inf and Imm 37:166-171 (1982) describe mouse monoclonal antibodies against P. aeruginosa outer membrane antigens. Galloway et al, Inf and Imm 44:262-267 (1984) describe mouse monoclonal antibodies specific to exotoxin A. Copending U.S. Patent Application Serial No. 727, 514, filed 26 April 1985, describes human monoclonal antibodies to exotoxin A. With respect to exoenzyme S, only rabbit polyclonal antibodies have been reported. Nicas and Iglewski, Infection and Immunity 45:470-474 (1984) and Pseudomonas aeruginosa New Therapeutic Approaches from Basic Research, Eds:D. Speert and R. Hancock, p. 40-48 by Nicas and Iglewski (S. Karger:Basel, 1985). These reports indicate that the rabbit polyclonal antibodies were effective in controlling P. aeruginosa infection in a mouse burn model.

Disclosure of the Invention

The present invention provides monoclonal antibody specific to P. aeruginosa exoenzyme S.

Another aspect of the invention herein is a stable, permanent cell line which produces such antibody and progeny of the cell line.

In addition the invention relates to compositions for treating infections caused by P. aeruginosa comprising a therapeutically effective amount of such an antibody that neutralizes the adverse biological effects of P. aerugenosa exoenzyme S in association with a pharmaceutically acceptable parenteral vehicle. In one embodiment the infection is chronic endobronchitis endemic in cystic fibrosis patients.

In a still further aspect the invention relates to the stable, permanent hybrid cell lines having ATCC accession numbers HB 9012 and HB 9013, and progeny of said cell lines.

The antibodies herein may be successfully utilized for passive immunotherapy against, or prophylaxis of, P. aeruginosa infections and for detection of P. aeruginosa exoenzyme S in samples suspected of containing the exoenzyme.

Modes of Carrying Out the Invention

As used herein the term "cell line" refers to individual cells, harvested cells, and cultures containing cells so long as they are derived from cells of the cell line referred to.

As used herein with respect to hybrid cell lines, the term "progeny" is intended to include all derivatives, issue, and offspring of the cell lines regardless of generation or karyotypic identity.

As used herein with respect to characterizing the claimed hybrid cell lines, the terms "permanent" and "stable" mean that the lines remain viable over a prolonged period of time, typically at least about six months, and maintain the ability to produce the specified monoclonal antibody through at least about 50 passages.

As used herein the term "monoclonal antibody" refers to an immunoglobulin composition having a substantially homogeneous population of antibodies, each of which binds to the same antigenic determinant. Unless otherwise indicated, the term is not intended to be limited to antibodies of any particular mammalian species or isotype or to antibodies prepared in any given manner. The term is intended to include whole antibody molecules as well as antigen-binding fragments (e.g., Fab, F(ab')$_2$, Fv).

As used herein with respect to a given antibody, the term "functional equivalent" means an antibody that recognizes the same determinant as and crossblocks the antibody referred to. It is intended to include antibodies of the same or different immunoglobin class and antigen binding fragments (e.g., Fab, F(ab')$_2$ Fv) of the antibody.

As used herein with respect to administering antibody to patients, the term "treat" and conjugates thereof refers to therapy and/or prophylaxis.

As used herein, the term "exoenzyme S" refers to either the unpurified form of exoenzyme S or to puri fied forms thereof such as the above-described 49 kd species, which are enzymatically active.

As used herein, the term "serotype" refers to one of the seven Fisher-Devlin-Gnabasik immunotypes of P. aeruginosa described by Fisher et al, J Bacteriol 98:833-836 (1969).

The anti-exoenzyme S monoclonal antibody of the invention will normally be of rodent or human origin because of the availability of murine, rat, and human tumor cell lines that may be used to produce immortal hybrid cell lines that secrete monoclonal antibody. The antibody may be of any isotype, but is preferably an IgG, IgM or IgA, most preferably an IgG2a.

A preferred procedure for making a hybrid cell line that secretes human anti-exoenzyme S is somatic cell hybridization using a mouse × human parent hybrid cell line and a human cell line producing sufficiently high levels of anti-exoenzyme S antibodies. The human cell line may be obtained from nonimmunized volunteers screened for or known to contain high serum titers of anti-exoenzyme S IgM, IgG and/or IgA. The human cell line may be transformed with Epstein-Barr virus (EBV) as described, for example, by Foung et al, J Immunol Methods 70:83-90 (1984).

When EBV transformation is employed the most successful approaches have been either to pre-select the population of B cells to be transformed or to post-select the antigen-specific transformed populations by panning or rosetting techniques, as described by Kozbar et al, Scan J Immunol 10:7-194 (1979) and Steinitz et al, J Clin Lab Immun 2:1-7 (1979). Recently EBV transformation has been combined with cell fusion to generate human monoclonal antibodies (see. e.g., Foung et al, J Immun Meth 70:83-90 (1984)), due to instability of im munoglobulin secretion by hybridomas when compared to EBV lymphoblastoid cell lines, and higher frequencies of rescue of the antigen-specific populations. EBV most frequently infects and transforms IgM-bearing B cells, but B cells secreting other classes of Ig can also be made into long-term lines using the EBV fusion technique, as described by Brown and Miller, J Immunol 128: 24-29 (1982).

Strategies for preparing and identifying hybridomas which produce murine anti-exoenzyme S antibodies of the invention include the following. Exoenzyme S is obtained and purified as described by Thompson et al in Novel ADP-Ribosylations of Regulatory Enzymes and Proteins, Smulson, Sugimura, Eds, Elsevier North Holland, Inc:1980 PP 425-433. The antibody-producing fusion partners which are used to make the hybridoma are produced by injecting a mouse with the exoenzyme S so obtained. The host animal is inoculated intraperitoneally with an immunogenic amount of the exoenzyme and then boosted periodically with similar amounts of the exoenzyme. Spleens or lymphoid tissue is collected from the immunized mice a few days after the final boost and a cell suspension is prepared therefrom for use in the fusion.

Hybridomas are prepared from the splenocytes or lymphoid tissue and a tumor (myeloma) partner using the general somatic cell hybridization technique of Milstein et al, Nature 256:495-497 (1975) and Koehler et al, Eur J Immunol 6:511-519 (1976). preferred myeloma cells for this purpose are those which fuse efficiently, support stable high-level expression of antibody by the selected antibody-producing cells, and are sensitive to a medium such as HAT medium. Among these, preferred myeloma cell lines are murine myeloma lines such as those derived from MOPC-21 and MOPC-11 mouse tumors available from the Salk Institute, Cell Distribution Center, San Diego, CA, or P3 × 63-Ag8.653 (653) and Sp2/0-Ag 14 (SP 2/0) myeloma lines available from the American Type Culture Collection, Rockville, MD, under ATCC CRL Nos. 1580 and 1581, respectively.

Basically, the technique involves fusing the appropriate tumor cells and splenocytes or lymphoid tissue using a fusogen such as polyethylene glycol. After the fusion the cells are separated from the fusion medium and grown on a selective growth medium, such as HAT medium, to eliminate unhybridized parent cells and to select only those hybridomas that are resistant to the medium and immortal. The hybridomas are expanded, if desired, and supernatants are assayed for exoenzyme S activity by conventional immunoassay procedures (e.g., radioimmunoassay enzyme immunoassay, or fluorescence immunoassay) using the immunizing agent as antigen.

The antigen-binding ability of the antibodies is evaluated in vitro by immunoblots, ELISAs and antigen neutralizing tests and in vivo using the mouse burn model. Those antibodies which have the ability to neutralize the adverse biological effects of P. aeruginosa exoenzyme S in mammals regardless of the mechanism involved are preferred.

The cell lines which produce the anti-exoenzyme S monoclonal antibodies may be grown in suitable culture medium such as Iscove's medium, Dulbecco's Modified Eagle's Medium, or RPMI-1640 medium from Gibco, Grand Island, NY, or in vivo in syngeneic or immunodeficient laboratory animals. If desired, the antibody may be separated from the culture medium or body fluid, as the case may be by conventional techniques such a ammonium sulfate precipitation hydroxylapatite chromatography, ion exchange chromatography, affinity chromatography, electrophoresis, microfiltration, and ultracentrifugation.

The antibodies of this invention that inactivate exoenzyme S may be used passively to treat individuals who

**0 243 174**

suffer from <u>P. aeruginosa</u> septicemia or are at risk with respect to <u>P. aeruginosa</u> infection. Patients at risk include those receiving immunosuppressive therapy and those suffering from severe thermal burns or other serious injuries, cystic fibrosis and cancer. One possible treatment is for chronic endobronchitic infection endemic in cystic fibrosis patients. While the antibodies will typically be used to treat humans, they may also be used to treat other mammalian species such as domestic and farm animals and sports or pet animals.

The antibodies may be administered to the patient by any suitable technique, including subcutaneous and parenteral administration, preferably parenteral. Examples of parenteral administration include intravenous, intraarterial, intramuscular and intraperitoneal. Intravenous administration is preferred. The dose and dosage regimen will depend mainly upon whether the antibody is being administered for therapeutic or prophylactic purposes, the patient, and the patient's history. The total pharmaceutically effective amount of an antibody administered per dose will typically be in the range of about 0.01 to 1 mg/kg of patient body weight.

For parenteral administration the antibody will generally be formulated in a unit dosage injectable form (solution, suspension, emulsion) in association with a pharmaceutically acceptable parenteral vehicle. Such vehicles are inherently nontoxic and nontherapeutic. Examples of such vehicles include water, saline, Ringer's solution, dextrose solution, and 5% human serum albumin. Nonaqueous vehicles such as fixed oils and ethyl oleate may also be used. Liposomes may be used as carriers. The vehicle may contain minor amounts of additives such as substances which enhance isotonicity and chemical stability, e.g., buffers and preservatives. The antibody will typically be formulated in such vehicles at a concentration of about 0.1 mg/ml to 100 mg/ml.

The anti-exoenzyme S monoclonal antibodies (whether neutralizing or nonneutralizing) may also be used to detect the presence or absence of exoenzyme S in body fluids such as blood serum, urine etc. or tissue cultures such as bacterial or animal cultures. The culture or body fluid may be incubated in the presence of the anti-exoenzyme S monoclonal antibody and the presence or absence and/or degree of reaction (antibody-exoenzyme S binding) can be determined by any of a variety of methods used to determine or quantitate antibody/antigen interactions (e.g., hemagglutination, latex agglutination, complement fixation, radioimmunoassay, enzyme immunoassay, fluorescent immunoassay, sandwich assay, fluorescent microscopy, etc). The incubation will be carried out under conditions that favor antibody/antigen reaction (i.e., physiological pH, temperature, and ionic strength). For example, a sandwich immunoassay may be used wherein the test sample is incubated with a first monoclonal antibody directed against one epitope of the antigen which is immobilized on a solid support such as a plastic tube or polystyrene beads and the test sample is incubated with a second monoclonal antibody directed against a different epitope of the antigen which is labeled with a detectable moiety which can be detected for example, immunologically, enzymatically, by use of a peptide or by spectrophotometric, chemical or radiological means. The incubation with the immobilized antibody may take place before, during or after incubation with the labeled antibody. Alternatively, the second antibody may be detected indirectly by reaction with a detectable ligand that binds to the second antibody.

For use in solid phase immunoassays, the antibodies herein may be conjugated to solid supports such as polystyrene beads using, for example, 2-(N-morpholino)ethanesulfonic acid buffer and 3-(3-dimethylaminopropyl)carbodiimide. Enzyme labels such as alkaline phosphatase or horseradish peroxidase may be conjugated to the antibody using coupling agents such as N-succinimidyl-3-(2-pyridyldithio)propionate (SPDP). These enzyme conjugated antibodies may then be used in an enzyme immunoassay such as that described by U.S. Patent 4,376,110.

The various aspects of the invention are further described by the following examples, which are not intended to limit the invention in any manner. In these examples all percentages for solids are by weight and all percentages for liquids and gases are by volume unless otherwise noted, and all temperatures are given in degrees Celsius.


Example 1


A. Preparation of Antibodies

Murine hybridomas that secrete monoclonal antibodies specific to exoenzyme S were prepared essentially by a modification of the fusion method of Oi and Herzenberg, Selected Methods in Cellular Immunology (Mishel and Shiigi, Eds), 1980.

Balb/c mice purchased from Charles River Lab, Cambridge, MA, were initially injected intraperitoneally with a mixture of 20 µg of purified exoenzyme S in phosphate-buffered saline (PBS) with 0.1% sodium dodecyl sulfate (SDS) and an equal volume of Freund's adjuvant complete (commercially obtained). After 2 weeks the mice were injected intraperitoneally with a mixture of 20 µg of purified exoenzyme S in PBS with 0.1% SDS and an equal volume of Freund's adjuvant incomplete. The mice were then injected with 10-20 µg of exoenzyme S in PBS with 0.1% SDS at several weekly intervals thereafter up to about 110 days after the initial injection. The last injection consisted of 10 µg of purified exoenzyme S in PBS with 0.1% SDS given intravenously. Three days after the final injection the mice were sacrificed and their spleens were removed.

The spleens were suspended in and washed three times with serum-free Dulbecco's Modified Eagle's Medium (DMEM) at room temperature. 653 or SP2/0 myeloma cells were harvested and washed three times with serum-free DMEM at room temperature. The immune spleen cells and fusion partners were mixed together at a ratio of 5:1 by cell number in a 50 ml centrifuge tube and centrifuged at 400 × g for 10 minutes at room temperature to form a tight pellet. All the supernate was removed from the pellet and the tube was

4

maintained at 37°C in a water bath.

One ml of warm 40% polyethylene glycol of molecular weight 1000 was added to the pellet over a one-minute period with gentle stirring. The pellet was then stirred for an additional minute. Then I ml of serum free DMEM warmed to 37°C was added over one minute, and this step was repeated.

Finally a total of 7 ml of serum-free DMEM warmed to 37°C was added over 2-3 minutes with continuous stirring. The resulting suspension was centrifuged at $400 \times g$ for 10 minutes at room temperature and the supernate was removed. A total of 10 ml of DMEM containing 20% fetal calf serum (FCS) warmed to 37°C was added to the cell pellet with stirring to form a suspension. An additional 20 ml of DMEM with 20% FCS warmed to 37°C was added with stirring. Cell density was counted with a hemacytometer and the cell concentration was adjusted with warm DMEM containing 20% FCS to $2 \times 10^6$ viable cells/ml.

A total of 0.1 ml of this suspension ($2 \times 10^5$ total cells) was plated into each well of 96-well tissue culture plates, called master plates, at fusion day 0. The plates were placed in a 6% $CO_2$-in-air incubator at 37°C. On day 1 clones were selected using 100 µl/well selection medium containing $2 \times$ azaserine and hypoxanthine ($2.2 \times 10^{-5}$ M and $2.0 \times 10^{-4}$ M azaserine and hypoxanthine respectively). The plates were aspirated and refed with selection medium containing $1 \times$ azaserine and hypoxanthine ($1.1 \times 10^{-5}$ M and $1.0 \times 10^{-4}$ M, respectively) on days 4 and 7. The supernates were assayed beginning on days 10-14, and feeding and assaying were continued up to about day 50.

### B. Screening with Exoenzyme S

A total of 500 µg of purified exoenzyme S (150 µg/ml) in 50 mM Tris, 384 mM glycine, 0.1% SDS, 1% NP40, and 1% 2-mercaptoethanol at pH 8.8 was diluted to a concentration of 100 ng/ml in 50 mM sodium carbonate buffer at pH 9.6, and coated onto 96-well flat-bottom microtiter plates at 100 µl/well (10 ng/well). After overnight incubation at 4°C, the plates were washed with PBS containing 0.1 g/l $MgCl_2$ and 0.1 g/l $CaCl_2$ (PBS++), 0.05% Tween 20 surfactant, and 0.01% Thimerosal.

After washing 200 µl of 50 mM sodium carbonate buffer plus 1% bovine serum albumin (BSA) at pH 9.6 was added to each well and the plates were incubated at 37°C for one hour. The plates were washed as described above and 100 µl of supernatant was added to each well and the plates were incubated at 37°C for one hour. Plates were washed again as described above and 100 µl of horseradish peroxidase-conjugated goat or rabbit anti-murine Ig developing reagents were added to each well and the plates were incubated at room temperature for 30 minutes. Plates were rinsed for a final time as described above and 200 µl of ABTS substrate (55 mg/ml of ABTS aqueous stock solution diluted 1:100 with 0.1 M sodium citrate buffer at pH 4.5 to which 30% $H_2O_2$ at 1:1000 was added immediately prior to use) was added to each well. The plates were developed in the dark at 37°C for 30 minutes and read with an ELISA reader at 405 nm. Readings were reported on a scale of 1 to 10 with 1 = 0.0 OD, 10 = 2.0 OD.

### C. Hybrid Screening

Culture supernatants were assayed by exoenzyme S ELISA as described in Section B of this example. All parent lines selected for antibody production yielded positive anti-exoenzyme S-secreting progeny. Most of the clones of all lines made appreciable amounts of specific antibody. For selecting of high-producer hybrids parental hybridomas were cloned by limiting dilution and progeny derived from a single cell were assayed for levels of reactive antibody.

### D. Immunoblotting (Western Blots)

Analytical polyacrylamide slab gel electrophoresis was carried out. The gels were 14 cm $\times$ 12 cm $\times$ 1.2 mm, 7-1/2% acrylamide in the separating gel, and were run at 0.45 mamp constant current. For protein staining the gel was placed in 25% methanol with 10% acetic acid and 0.25% Coomassie Blue R-250 at 50°C for 15 min and destained in 10% methanol plus 10% acetic acid.

For the Western blot, purified 49 kd exoenzyme S was transferred onto a sheet of nitrocellulose of 0.45 µm pore size (55 volts for 2 hr). The nitrocellulose sheet was soaked for 30 min in 10 mM Tris pH 7.4, containing 150 mM NaCl and 1% BSA (buffer A). The sheet was rinsed with deionized water and incubated for 18 hr at 4°C with an equal volume of buffer A and anti-exoenzyme S monoclonal antibody tissue culture supernatant. The sheet was then rinsed again and incubated for two hr at 25°C with buffer A containing a 1000-fold dilution of horseradish peroxidase conjugated goat anti-mouse IgG (heavy and light chain specific). The sheet was rinsed and incubated for 30 min at 25°C with buffer A containing 0.05% Triton X-100 in place of the BSA. The sheet was rinsed and the detergent wash was repeated. The sheet was rinsed and placed in 60 ml of 10 mM Tris pH 7.4, by adding 20 ml of methanol containing 60 mg of 4-chloronaphthol and 60 µl of 30% hydrogen peroxide. The sheet in this solution was agitated at 25°C and the reaction was terminated by rinsing with cold water containing 10 mM sodium azide.

Four of the antibodies (designated 4F7, 1F6, 10F10, and 15D4) selected in the hybrid screening showed binding to the purified 49 kd exoenzyme S in the blots. 10Fl0 was identified as an IgGl and a switch variant IgG2a and 15D4, 4F7 and 1F6 were identified as IgGl antibodies.

### Enzyme Neutralization Tests

Purified 49kd exoenzyme S was diluted in blank tissue culture medium to a concentration of 1 µg/ml and 10 µl of this was placed in a 1.5 ml polypropylene microfuge tube. An equal volume of monoclonal tissue culture supernatant was added and the mixture was incubated at 4°C for 18 hr. The samples were then assayed in a standard exoenzyme S assay. The amount of exoenzyme S used (1.25 ng per assay) and the length of time of the assay were such as to be in the linear response range of the enzyme's activity. All of the assays were done in duplicate and the entire neutralization experiment was performed twice (designated Exp 1 and Exp 2 in the following table).

The enzyme assays were performed as follows. Enzyme sample, 2.5 µl, was mixed with 25 µl of 200 mM sodium acetate, pH 6.0, and 25 µl of a wheat germ extract and then held at 25°C for 1 min. The reaction was started by adding 5 µl of 17 µ molar (adenosine $^{14}$C (U)) NAD (540 mCi/mmole) and terminated after 5 min with 100 µl of 10% trichloroacetic acid (TCA). The precipitate was collected on a 25 mm cellulose acetate filter, washed with 5% TCA, and counted in a low background counter. The results of these tests are tabulated below.

| Sample | Experiment 1 | | Experiment 2 | |
|---|---|---|---|---|
| | cpm | % Inhibition | cpm | % Inhibition |
| Blank | 48 | – | 70 | – |
| 49kd Exo S Control[1] | 478 | 0 | 540 | 0 |
| Neutralizing antibody | | | | |
| anti-49kd rabbit polyclonal[2] | 57 | 98% | 55 | 100% |
| 4F7 | 365 | 34% | 392 | 31% |
| 1F6 | 352 | 36% | 381 | 34% |
| 10F10 | 202 | 68% | 300 | 51% |
| 15D4 | 510 | 0 | 546 | 0 |

[1] 49K Exo S control was incubated with blank tissue culture medium.

[2] Purifed IgG at 9 mg/ml.

The lack of neutralization exhibited by 15D4 was believed to be due to the fact that supernate of unknown titer was used in the test.

Enzyme neutralization assays were then performed with purified 1F6, 10F10 (IgG1). 10F10 (IgG2a) and 15D4 anti-exoenzyme S antibodies. Isotype-specific murine monoclonal antibodies known to be non-reactive with 49 kd exoenzyme S were used as negative controls. The conditions of neutralization and assay were the same as above, except the diluting solution was phosphate buffered saline (PBS) pH 7.4 with 0.1% Triton X-100 (membrane grade from Boehringer Mannheim) rather than tissue culture medium. The concentration of exoenzyme S during neutralization was 0.5 µg/ml and the concentrations of antibody were 15, 5, 1.5, 0.5 or 0.15 µg/ml. The counts per minute (cpm) value listed is the average of duplicate assays and are corrected for a blank con trol of 47 cpm. The results of these tests are tabulated below:

| Sample | µg Antibody/ml | cpm | % Inhibition |
|---|---|---|---|
| Blank | ---- | 47 | ---- |
| 49kd Exo S Control[1] | ---- | 923 | ---- |
| Isotype specific controls[2] | | | |
| IgG1[3] | ---- | 853 | 8 |
| IgG2a[4] | ---- | 898 | 3 |
| IgG2b[5] | ---- | 904 | 2 |
| Anti-exoenzyme S antibodies | | | |
| 1F6 | 15 | 561 | 39 |
| | 5 | 589 | 36 |
| | 1.5 | 813 | 12 |
| | 0.5 | 835 | 10 |
| | 0.15 | 906 | 2 |
| 10F10 (IgG1) | 15 | 54 | 94 |
| | 5 | 55 | 94 |
| | 1.5 | 280 | 70 |
| | 0.5 | 675 | 27 |
| | 0.15 | 892 | 3 |
| 10F10 (IgG2a) | 15 | 34 | 96 |
| | 5 | 39 | 96 |
| | 1.5 | 115 | 88 |
| | 0.5 | 586 | 37 |
| | 0.15 | 825 | 11 |

| Sample | µg Anti-body/ml | cpm | % Inhibition |
|--------|-----------------|-----|--------------|
| 15D4 | 15 | 769 | 17 |
| | 5 | 812 | 12 |
| | 1.5 | 903 | 2 |
| | 0.5 | 879 | 5 |
| | 0.15 | 833 | 10 |

[1]49kd Exo S control was incubated with PBS pH 7.4 with 0.1% Triton X-100.

[2]Average of all five concentrations.

[3]T3-3A1 murine monoclonal against human $T_4$ lymphocyte receptor.

[4]77-20 8H12 1E8 1D4 murine monoclonal against interleukin-2.

[5]OKT4 murine monoclonal against human $T_4$ lymphocyte receptor.

Deposits

Samples of two of the hybridomas that secrete anti-exoenzyme S antibodies 10F10 and 15D4 were deposited at the Cetus Tissue Culture Collection of the coassignee (CTCC) and at the American Type Culture Collection (ATCC), 12301 Parklawn Drive, Rockville, MD, U.S.A. ATCC deposit dates and ATCC accession numbers are given below for these lines.

| Cell Line Designation | ATCC Deposit Date | ATCC Accession No. |
|-----------------------|-------------------|--------------------|
| 10F10 | 5 February 1986 | HB 9013 |
| 15D4 | 5 February 1986 | HB 9012 |

These ATCC deposits were made under the provisions of the Budapest Treaty on the International Recognition of the Deposit of Microorganisms for the Purposes of Patent Procedure and the Regulations thereunder (Budapest Treaty). Availability of the deposited cultures is not to be construed as a license to practice the invention in contravention of the rights granted under the authority of any government in accordance with its patent laws.

The deposit of these cell lines does not constitute an admission that the written description of this application is inadequate to enable the practice of any aspect of the invention, nor is it to be construed as limiting the scope of the claims to the specific illustrations which these lines represent.

**Claims**

1. A monoclonal antibody to Pseudomonas aeruginosa exoenzyme S.
2. The antibody of claim 1 which is of the isotype IgA IgG or IgM.
3. The antibody of claim 1 or 2 which is murine or human.
4. The antibody of claim 1, 2 or 3 which neutralizes the adverse biological effects of said exoenzyme S.

5. The antibody of claim 1 wherein the antibody is produced by hybridoma HB 9012.

6. The antibody of claim 1 wherein the antibody is produced by hybridoma HB 9013.

7. A stable, permanent cell line which produces the antibody of claim I, 2, 3 or 4 and progeny thereof.

8. The cell line of claim 7 which is HB 9012 or HB 9013.

9. A composition for treating infections caused by Pseudomonas aeruginosa comprising a therapeutically effective amount of the antibody of claim 4 or 6 in association with a pharmaceutically acceptable parenteral vehicle.

10. A method of detecting the presence or absence of Pseudomonas aeruginosa exoenzyme S in a sample suspected of containing the exoenzyme S comprising incubating the sample in the presence of the antibody of claim 1, 2, 3 4, 5 or 6 which is labeled with a detectable moiety and detecting the moiety.

11. A method of detecting the presence or absence of Pseudomonas aeruginosa exoenzyme S in a sample suspected of containing said exoenzyme S comprising incubating the sample with the antibody of claim I, 2, 3, 4, 5 or 6 and determining whether the antibody has reacted with the sample and/or the extent to which the antibody has reacted with the sample.

12. A method of detecting the presence or absence of Pseudomonas aeruginosa exoenzyme S in a sample suspected of containing said exoenzyme S comprising incubating the sample with the antibody of claim 1, 2, 3, 4, 5 or 6 and incubating any resulting immune-complexes formed during the incubation with a detectable ligand reactive with the antibody and detecting ligand-antibody complexes.